# EUROPEAN PATENT APPLICATION

(11) **EP 2 302 414 A2**
(43) Date of publication of application: **30.03.2011**
(21) Application number: 10175435.6
(22) Date of filing: 06.09.2010
(51) Int. Cl.: G01S 15/89, G06T 7/60, A61B 8/00

(54) **Ultrasound system and method of performing measurement on three-dimensional ultrasound image**

(30) Priority: 16.09.2009 KR 20090087394
(71) Applicant: MEDISON CO., LTD., Gangwon-do 250-875 (KR)
(72) Inventor: Lee, Kwang Hee, Seoul 135-851 (KR); Lee, Ki Jong, Seoul 135-851 (KR); Kim, Sung Yoon, Seoul 135-851 (KR)
(74) Representative: Schmid, Wolfgang

(57) **Abstract**

Embodiments for providing an ultrasound system of performing a 3D measurement, comprise: an ultrasound data acquisition unit configured to transmit ultrasound signals to a target object and receive ultrasound echo signals reflected from the target object to acquire ultrasound data; a user interface configured to receive input data from a user; and a processor configured to form a 3D-ultrasound image based on volume data derived from the ultrasound data, establish two or more points on the 3D-ultrasound image based on the input data, generate connection data among the established two or more points on the 3D-ultrasound image, and measure distances among the established two or more points based on the input data and the connection data.

## Description

### TECHNICAL FIELD

The present disclosure generally relates to an ultrasound system, and more particularly to an ultrasound system and method of performing a measurement for points established on a three-dimensional (3D)-ultrasound image.

### BACKGROUND

An ultrasound diagnostic system has been extensively used in the medical field due to its non-invasive and non-destructive nature. The ultrasound diagnostic system can provide a high-resolution ultrasound image of the inside of a target object in real-time without resorting to any incisions.

A three-dimensional (3D) ultrasound image from the ultrasound diagnostic system may include clinical data such as spatial coordinates data, anatomical geometry and the like, which may not be sufficiently provided from a two-dimensional (2D) ultrasound image. Thus, the 3D-ultrasound image may be used in the medical field during diagnosis and surgical operations. Conventionally, at least two points may be established on a same 2D slice image of a 3D-ultrasound image and a distance between the two points may then be measured. However, if two or more points are established on different 2D slice images of the 3D-ultrasound image, then distances among the established points on the different 2D slice images may not be measured.

### SUMMARY

Embodiments for providing an ultrasound system and a method of performing a three-dimensional (3D) measurement for points established on a 3D-ultrasound image are provided. In accordance with one embodiment of the present disclosure, there is provided an ultrasound system of performing a 3D measurement, which comprises: an ultrasound data acquisition unit configured to transmit ultrasound signals to a target object and receive ultrasound echo signals reflected from the target object to acquire ultrasound data; a user interface configured to receive input data from a user; and a processor configured to form a 3D-ultrasound image based on volume data derived from the ultrasound data, establish two or more points on the 3D-ultrasound image based on the input data, generate connection data among the established two or more points on the 3D-ultrasound image, and measure distances among the established two or more points based on the input data and the connection data.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

- FIG. 1: is a block diagram showing an ultrasound system in accordance with one embodiment of the present disclosure.
- FIG. 2: is a block diagram showing an ultrasound data acquisition unit in the ultrasound system in accordance with one embodiment of the present disclosure.
- FIG. 3: is a block diagram showing a processor in the ultrasound system in accordance with one embodiment of the present disclosure.
- FIG. 4: is a flow chart showing a process in the ultrasound system in accordance with one embodiment of the present disclosure.
- FIG. 5: is a schematic diagram showing a scan direction of 2D slice images of a 3D- ultrasound image in accordance with the present disclosure.
- FIG. 6: is an illustrative embodiment showing volume data in accordance with one embodiment of the present disclosure.
- FIG. 7: is an illustrative embodiment showing 2D slice images corresponding to cross- section planes A to C crossed at right angles in accordance with one embodiment of the present disclosure.
- FIG. 8: is an illustrative embodiment showing points established on the 2D slice images according to input data in accordance with one embodiment of the present disclosure.
- FIG. 9: is an illustrative embodiment showing points established on a 3D-ultrasound image according to the input data in accordance with one embodiment of the present disclosure.
- FIG. 10: is an illustrative embodiment showing connection data among the points established on the 3D-ultrasound image in accordance with one embodiment of the present disclosure.
- FIG. 11: is an illustrative embodiment showing distance measurement data among the points established on the 3D-ultrasound image in accordance with one embodiment of the present disclosure.

### DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

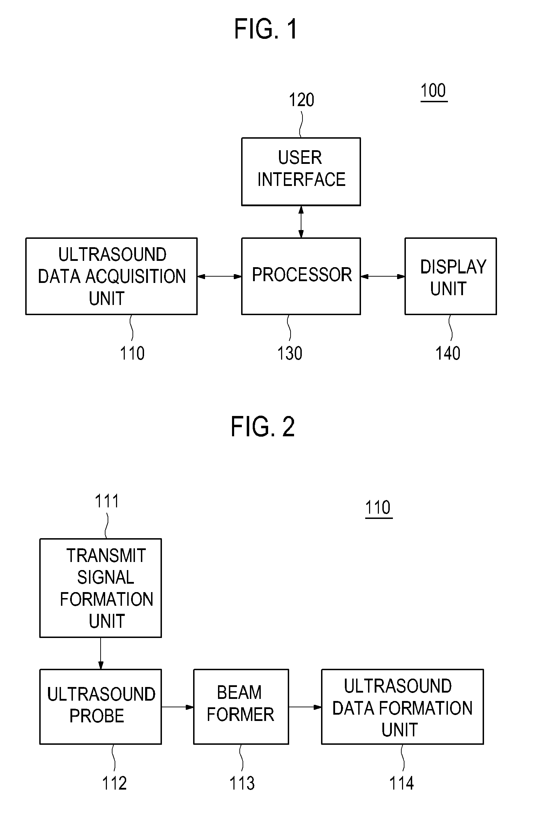

Referring to FIG. 1, there is shown a block diagram illustrating an ultrasound system 100 in accordance with one embodiment of the present disclosure. The ultrasound system 100 may comprise an ultrasound data acquisition unit 110, a user interface 120, a processor 130 and a display unit 140.

The ultrasound data acquisition unit 110 may be configured to transmit ultrasound signals to a target object and receive reflected ultrasound signals, i.e., ultrasound echo signals, from the target object to acquire ultrasound data thereof. The organization of the ultrasound data acquisition unit 110 will be described later with reference to FIG. 2.

The user interface 120 may be configured to receive input data from a user. The user interface 120 may include a control panel, a mouse, a keyboard and a touch screen on which ultrasound images are displayed. However, it is not limited thereto. The input data from the user may include data for points to be established on regions of interest (ROIs) of a 3D-ultrasound image of the target object and/or of different 2D slice images thereof. In one embodiment, the input data may include 3D and/or 2D coordinate values of the points to be established, the number of points to be established, and/or data related to 2D slice images designated by the user for establishing the points.

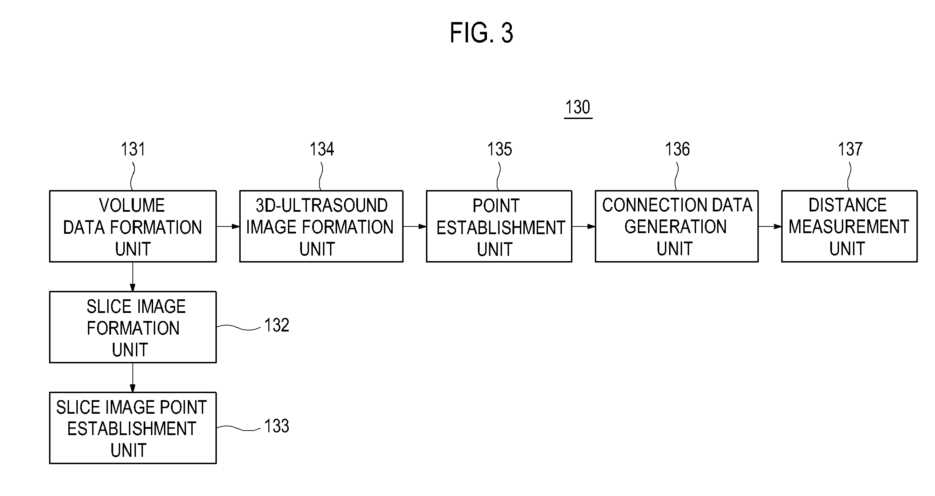

The processor 130 may be configured to form the 3D-ultrasound image and 2D slice images thereof based on the ultrasound data from the ultrasound data acquisition unit 110. In response to the input data, the processor 130 may be configured to establish two or more points on the 3D-ultrasound image and the 2D slice images thereof, and further provide connection data and distance measurement data for the established points. The detailed description for organization and operation of the processor 130 will be made later with reference to FIG. 3.

The display unit 140 may be configured to display the 3D-ultrasound image and/or the 2D slice images thereof. The display unit 140 may be further configured to display the connection data and/or the distance measurement data over the 3D-ultrasound image and/or the 2D slice images thereof.

Referring now to FIG. 2, there is shown a block diagram showing the ultrasound data acquisition unit 110 in the ultrasound system 100 in accordance with one embodiment of the present disclosure. The ultrasound data acquisition unit 110 may comprise a transmit signal formation unit 111, an ultrasound probe 112 having a plurality of transducer elements (not shown), a beam former 113 and an ultrasound data formation unit 114.

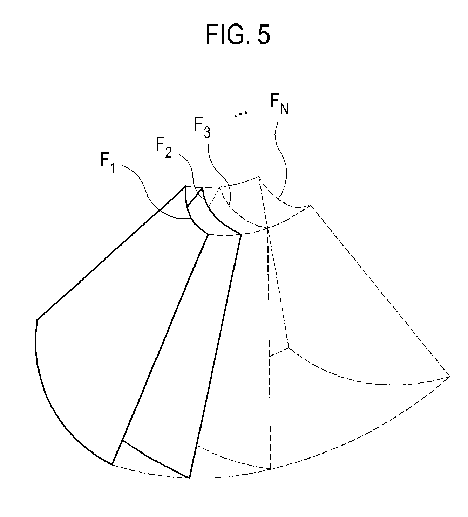

The transmit signal formation unit 111 may be configured to form the transmit signals for obtaining a series of 2D slice images Fᵢ (1≤i≤N, N being an integer) shown in FIG. 5 in consideration of positions and focusing points of the transducer elements in the ultrasound probe 112. The series of 2D slice images Fᵢ are represented in the form of a fan-shaped image as shown in FIG. 5, although they are not limited thereto.

In response to the transmit signals from the transmit signal formation unit 111, the ultrasound probe 112 may be configured to convert the transmit signals into corresponding ultrasound signals and transmit them to the target object. The ultrasound probe 112 may be further configured to receive ultrasound echo signals reflected from the target object to form receive signals. In an exemplary embodiment, the ultrasound probe 112 may include at least one of a 3D probe and 2D-arrayed probe.

In response to the receive signals from the ultrasound probe 112, the beam former 113 may be configured to convert the receive signals from analog into digital to form digital signals corresponding thereto. The beam former 113 may be further configured to receive-focus the digital signals in consideration of the positions and focusing points of the transducer elements in the ultrasound probe 112 to form a receive-focus beam.

The ultrasound data formation unit 114 may be configured to form ultrasound data based on the receive-focus beam. In one embodiment, the ultrasound data formation unit 114 may be configured to perform various signal processes (e.g., gain adjustment, filtering, etc.) upon the receive-focus beam in order to form the ultrasound data.

Referring to FIG. 3, there is provided a block diagram showing the processor 130 in the ultrasound system 100 in accordance with one embodiment of the present disclosure. The processor 130 may include a volume data formation unit 131, a slice image formation unit 132, a slice image point establishment unit 133, a 3D-ultrasound image formation unit 134, a point establishment unit 135, a connection data generation unit 136 and a distance measurement unit 137.

The volume data formation unit 131 may be configured to form volume data based on the ultrasound data from the ultrasound data acquisition unit 110, as shown in FIG. 1. The volume data may include a plurality of voxels having brightness values. The slice image formation unit 132 may be configured to form the 2D slice images of the target object based on the volume data from the volume data formation unit 131. The slice image point establishment unit 133 may be configured to establish two or more points on corresponding 2D slice images based on the input data from the user interface 120, as shown in FIG. 1. The corresponding 2D slice images may be different from each other.

The 3D-ultrasound image formation unit 134 may be configured to render the volume data from the volume data formation unit 131 to form the 3D-ultrasound image of the target object. The point establishment unit 135 may be configured to establish two or more points on the 3D-ultrasound image based on the input data from the user interface 120. The connection data generation unit 136 may be configured to generate connection data among the two or more points established on the 3D-ultrasound image. The connection data may indicate relative coordinate values among the established points. The connection data may be provided to the display unit 140, as shown in FIG. 1. The distance measurement unit 137 may be configured to measure distances among the established points based on the connection data from the connection data generation unit 136 to form distance measurement data. The distance measurement data may be provided to the display unit 140, as well as the connection data. The processor 130 may further include a movement estimation unit (not shown) for estimating movements of the established points on the 3D-ultrasound image and variations of the connection data by using, for example, cross correlation. In one exemplary embodiment, the movement estimation unit may calculate cross correlation at plural points on a subsequent 3D-ultrasound image centering on each of the established points on the current 3D-ultrasound image and select one out of the plural points having a maximum cross correlation with respect to the respective established points. Thereafter, the movement estimation unit may estimate movement of the established points and variations of the connection data based on the cross correlation result between the current and subsequent 3D-ultrasound images.

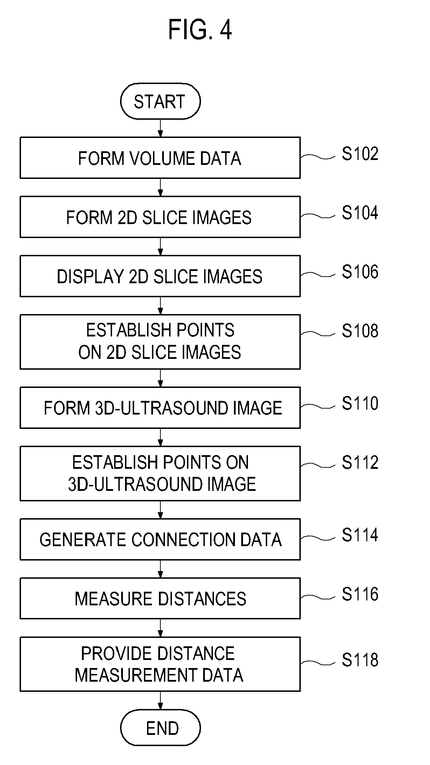

Hereinafter, operations of the processor 130 will be described in detail with reference to the accompanying drawings. Referring to FIG. 4, there is provided a flow chart showing a series of processes performed in the processor 130 in the ultrasound system 100 in accordance with one embodiment of the present disclosure.

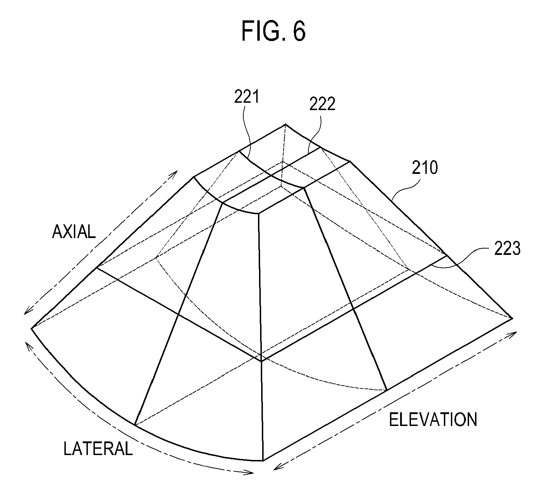

The volume data formation unit 131 may form volume data 210 shown in FIG. 6 based on the ultrasound data from the ultrasound data acquisition unit 110 (S102). In an exemplary embodiment, as shown in FIG. 6, reference numerals 221 to 223 indicate cross-sections A, B and C, which are crossed at right angles, respectively. Also, as shown in FIG. 6, an axial direction indicates a propagation direction of the ultrasound signals starting from the transducer elements of the ultrasound probe 112, a lateral direction represents a scan line direction of the ultrasound signals, and an elevation direction depicts a depth direction of the 3D-ultrasound image.

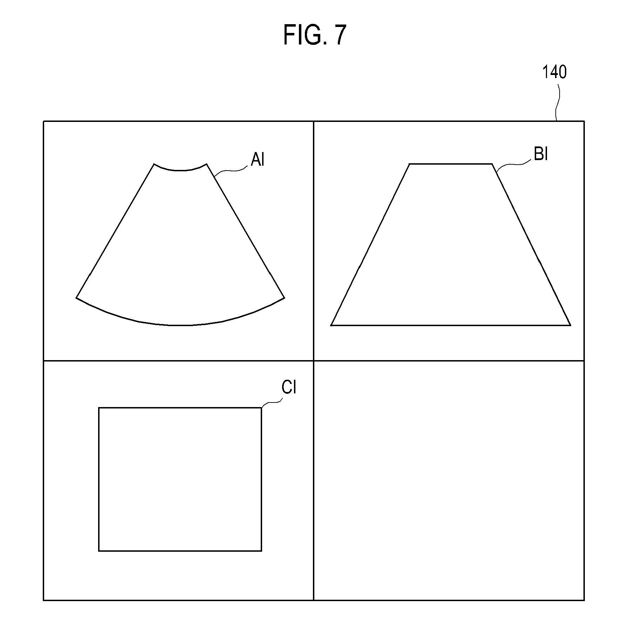

The slice image formation unit 132 may form a plurality of 2D slice images corresponding to cross-sections based on the volume data from the volume data formation unit 131 (S104). In one embodiment, the 2D slice images may include first to third 2D slice images AI, BI and CI shown in FIG. 7 corresponding to the cross-sections A, B and C 221 to 223, respectively. The first to third 2D slice images AI, BI and CI may be displayed through the display unit 140 in a predetermined arrangement form (S 106).

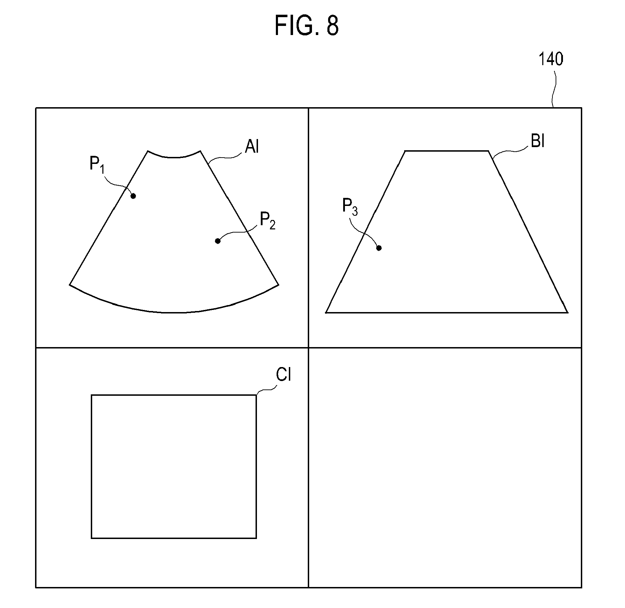

Based on the input data from the user interface 120, the slice image point establishment unit 133 may establish two or more points on the 2D slice images designated by the input data from the user interface 120 (S108). In an exemplary embodiment, the input data may include data related to first and second points P₁ and P₂ to be established on the first 2D slice image AI corresponding to the cross-section A 221, and a third point P3 to be established on the second 2D slice image BI corresponding to the cross-section B 222. Then, based on the input data, the slice image point establishment unit 133 may establish the first and second point P₁ and P₂ on the first 2D slice image AI, and the third point P₃ on the second 2D slice image BI, as shown in FIG. 8.

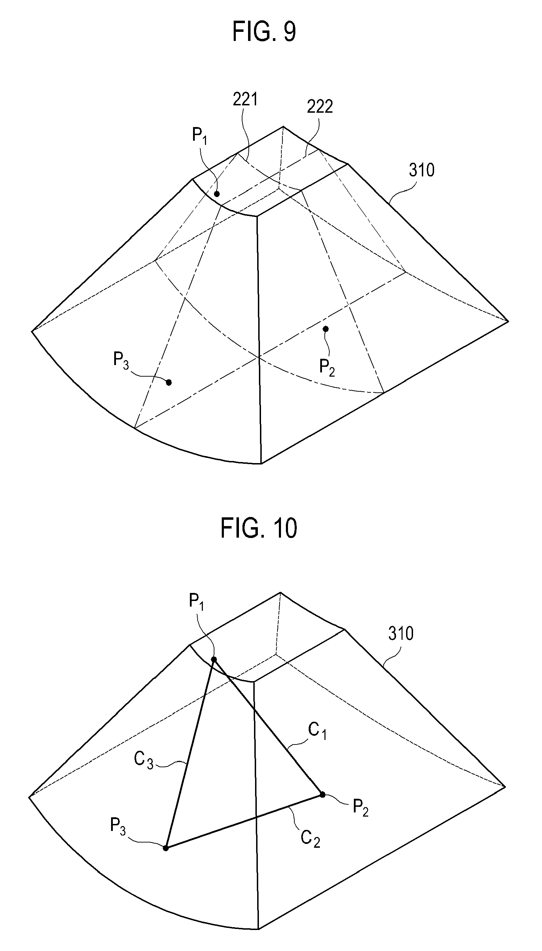

The 3D-ultrasound image formation unit 134 may render the volume data from the volume data formation unit 131 to form a 3D-ultrasound image 310 of the target object, as shown in FIG. 9 (S110). In an exemplary embodiment, rendering of the volume data in the 3D-ultrasound image formation unit 143 may include ray-casting rendering, surface rendering and the like.

The point establishment unit 135 may establish two or more points on the 3D-ultrasound image 310 from the 3D-ultrasound image formation unit 134 based on the input data (S 112). In an exemplary embodiment, as shown in FIG. 9, the point establishment unit 135 may establish the points P₁ and P₂ at corresponding positions of the cross-section A 221 of the 3D-ultrasound image 310, and the point P₃ at a corresponding position of the cross-section B 222 of the 3D-ultrasound image 310.

The connection data generation unit 136 may generate the connection data among the first to third points P₁ to P₃ established on the 3D-ultrasound image 310 and provide them to the display unit 140 (S 114). In an exemplary embodiment, as shown in FIG. 10, the connection data generation unit 136 may generate first connection data C₁ between the first and second points P₁ and P₂, second connection data C₂ between the second and third points P₂ and P₃, and third connection data C₃ between the first and third points P₁ and P₃. The display unit 140 may display the first to third connection data C₁ to C₃ in a predetermined arrangement form as shown in FIG. 10, although the displayed arrangement form is not limited thereto.

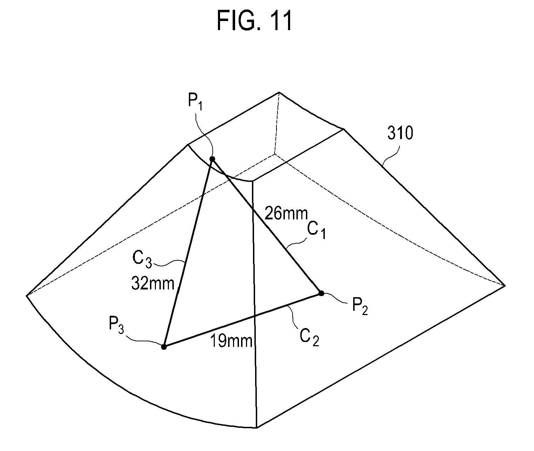

The distance measurement unit 137 may measure distances among the established points on the 3D-ultrasound image based on the input data and the connection data to thereby form distance measurement data (S 116). In an exemplary embodiment, as shown in FIG. 11, the distance measurement unit 137 may measure a distance between the first and second points P₁ and P₂ to form first distance measurement data (e.g., 26 mm), a distance between the second and third points P₂ and P₃ to form second distance measurement data (e.g., 19 mm), and a distance between the first and third points P₁ and P₃ to form third distance measurement data (e.g., 32 mm). The distance measurement data are described herein as numerical data, but they are not limited thereto. The distance measurement unit 137 may provide the distance measurement data to the display unit 140. Then, the display unit 140 may display the first to third distance measurement data thereon at predetermined positions and in predetermined forms.

Although exemplary embodiments have been described with reference to a number of illustrative embodiments thereof, it should be understood that numerous other modifications and embodiments can be devised by those skilled in the art that will fall within the scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
an ultrasound data acquisition unit configured to transmit ultrasound signals to a target object and receive ultrasound echo signals reflected from the target object to acquire ultrasound data;
a user interface configured to receive input data from a user; and
a processor configured to form a three-dimensional (3D) ultrasound image based on volume data derived from the ultrasound data,
establish two or more points on the 3D-ultrasound image based on the input data, generate connection data among the established two or more points on the 3D-ultrasound image, and measure distances among the established two or more points based on the input data and the connection data,
wherein the established two or more points exist on different cross-sections of the 3D-ultrasound image, and the cross-sections of the 3D-ultrasound image correspond to 2D slice images formed based on the volume data.

2. The ultrasound system of Claim 1, wherein the input data includes data related to coordinates values of the established two or more points, a number of the points and 2D slice images to be designated by the user.

3. The ultrasound system of Claim 2, wherein the processor includes:
a volume data formation unit configured to form the volume data based on the ultrasound data;
a 3D-ultrasound image formation unit configured to form the 3D-ultrasound image based on the volume data;
a point establishment unit configured to establish the two or more points on corresponding positions of the 3D-ultrasound image based on the input data;
a connection data generation unit configured to generate the connection data in consideration with a relationship among the established two or more points; and
a distance measurement unit configured to measure the distances among the established two or more points based on the input data and the connection data to generate distance measurement data.

4. The ultrasound system of Claim 2, wherein the processor further includes:
a slice image formation unit configured to form the 2D slice images corresponding to the cross-sections of the 3D-ultrasound image based on the volume data; and
a slice image point establishment unit configured to establish the two or more points on corresponding ones among the 2D slice images based on the input data.

5. The ultrasound system of Claim 2, further comprises a movement estimation unit configured to estimate movements of the established two or more points and variations of relative coordinates values among the established two or more points.

6. The ultrasound system of Claim 3, further comprises a display unit configured to display the established two or more points, the connection data, the distance measurement data and the 3D-ultrasound image,
wherein the established two or more points are displayed on the 3D-ultrasound image and at least one of the connection data, and wherein the distance measurement data and combination thereof are displayed on the 3D-ultrasound image together with the established two or more points.

7. The ultrasound system of Claim 4, further comprises a display unit configured to display the established two or more points and the 2D slice images designated by the user, wherein the established two or more points are displayed on the designated 2D slice images.

8. A method of measuring in an ultrasound system, comprising:
transmitting ultrasound signals to a target object and receiving ultrasound echo signals reflected from the target object to form ultrasound data;
forming volume data based on the ultrasound data;
receiving input data from a user;
forming a 3D-ultrasound image based on the volume data;
establishing two or more points on the 3D-ultrasound image based on the input data;
generating connection data among the established two or more points on the 3D-ultrasound image; and
measuring distances among the established two or more points based on the input data and the connection data to thereby generate distance measurement data,
wherein the established two or more points exist on different cross-sections of the 3D-ultrasound image, and the different cross-sections of the 3D-ultrasound image correspond to 2D slice images formed based on the volume data.

9. The method of Claim 8, wherein the input data includes data related to coordinates values of the established two or more points and a number of the points.

10. The method of Claim 9, wherein the input data further includes data related to 2D slice images to be designated by the user.

11. The method of Claim 9, further comprising, before receiving input data from a user, forming the 2D slice images corresponding to cross-sections of the 3D-ultrasound image based on the volume data.

12. The method of Claim 9, further comprising, before forming a 3D-ultrasound image based on the volume data, establishing the two or more points on corresponding 2D slice images based on the input data.

13. The method of Claim 8, further comprising estimating movements of the established two or more points on the 3D-ultrasound image.

14. The method of Claim 8, further comprising displaying the 3D-ultrasound image together with the connection data and distance measurement data related to the established two or more points.

15. The method of Claim 9, further comprising displaying the 2D slice images together with the established two or more points displayed on the 2D slice images.
